# EUROPEAN PATENT APPLICATION

(11) **EP 4 825 435 A1**
(43) Date of publication of application: **21.10.2026**
(21) Application number: 24903579.1
(22) Date of filing: 04.12.2024
(51) Int. Cl.: A61N 1/36

(54) **ELECTRIC TASTE PRESENTATION DEVICE**

(30) Priority: 11.12.2023 JP 2023208670
(71) Applicant: Ubeing Inc., Nagoya-shi, Aichi 466-0064 (JP)
(72) Inventor: FUKUSHIMA, Taiki, Nagoya-shi, Aichi 466-0064 (JP)
(74) Representative: Gulde & Partner
(86) International application number: PCT/JP2024/042846
(87) International publication number: WO 2025/126922

(57) **Abstract**

An object of the present invention is to provide an electric taste presentation device capable of suppressing the reduction of the taste presentation effect for a user. An electric taste presentation device includes an elongated first electrode that comes into contact with a first portion of a human jaw, an elongated second electrode that comes into contact with a second portion of the human jaw, and an electric signal generation unit that applies an electric signal between the first electrode and the second electrode. Alternatively, the electric taste presentation device includes a plurality of first electrodes that come into contact with a first portion of a human jaw, a plurality of second electrodes that come into contact with a second portion of the human jaw, and an electric signal generation unit that applies an electric signal between the plurality of first electrodes and the plurality of second electrodes. The electric taste presentation device presents electric taste to a human body by applying an electric signal to taste organs of the human body via the first electrode and the second electrode.

## Description

### Technical Field

The present invention relates to an electric taste presentation device.

### Background Art

Excessive intake of salt and sugar has been reported as one of the causes of health problems. On the other hand, a low-salt diet that reduces the amount of salt is recommended, but it generally reduces the sense of satisfaction from meals.

As a method for presenting a sufficient taste sensation to satisfy even a low-salt diet, which is considered "bland" and reduces satisfaction from meals, a technique has been disclosed for electrically presenting taste by applying electrical stimulation to a user's taste organs in the oral cavity.

For example, Patent Literature 1 discloses a taste electrical stimulation device in which electrodes for applying an electric signal are attached to a user's head. Furthermore, Patent Literature 2 discloses an electric taste presentation device in which electrodes are placed on a user's fingertips, and an electric signal is applied through conductive tableware such as chopsticks or forks and food.

### Citation List

### Patent Literature

[Patent Literature 1] Japanese Unexamined Patent Application Publication No. 2018-42991
[Patent Literature 2] Japanese Unexamined Patent Application Publication No. 2021-45399

### Summary of Invention

### Technical Problem

However, in the taste electrical stimulation device disclosed in Patent Literature 1 and the electric taste presentation device disclosed in Patent Literature 2, there was a problem that when applying electrical stimulation to the user's taste organs in the oral cavity, the user might experience pain or discomfort, which in turn could reduce the effect of taste presentation to the user.

An object of the present invention is to provide an electric taste presentation device capable of suppressing the reduction of the taste presentation effect for a user.

### Solution to Problem

According to the present invention, an electric taste presentation device with the following configuration is provided.
[1] An electric taste presentation device for presenting electric taste by electrical stimulation to a human body, comprising: an elongated first electrode that comes into contact with a first portion of a jaw of the human body; an elongated second electrode that comes into contact with a second portion of the jaw of the human body; and an electric signal generation unit that applies an electric signal between the first electrode and the second electrode, wherein the electric taste presentation device presents electric taste to the human body by applying the electric signal to taste organs of the human body via the first electrode and the second electrode.
[2] An electric taste presentation device for presenting electric taste by electrical stimulation to a human body, comprising: a plurality of first electrodes that come into contact with a first portion of a jaw of the human body; a plurality of second electrodes that come into contact with a second portion of the jaw of the human body; and an electric signal generation unit that applies an electric signal between the plurality of first electrodes and the plurality of second electrodes, wherein the electric taste presentation device presents electric taste to the human body by applying the electric signal to taste organs of the human body via the plurality of first electrodes and the plurality of second electrodes.

According to the present invention, by making the first electrode, which comes into contact with a first portion of the human jaw to apply an electric signal, and the second electrode, which comes into contact with a second portion of the human jaw, elongated, it is possible to disperse the application range of the electric signal to the jaw when applying electrical stimulation to the user's taste organs in the oral cavity, thereby suppressing the risk of the user experiencing pain or discomfort, and consequently suppressing the reduction of the taste presentation effect for the user. Furthermore, according to the present invention, by dividing the first electrode that comes into contact with the first portion of the human jaw into multiple electrodes and dividing the second electrode that comes into contact with the second portion of the human jaw into multiple electrodes, it is possible to disperse the application range of the electric signal to the jaw when applying electrical stimulation to the user's taste organs in the oral cavity, thereby suppressing the risk of the user experiencing pain or discomfort, and consequently suppressing the reduction of the taste presentation effect for the user.

Various embodiments of the present invention will be illustrated below. The embodiments shown below can be combined with each other.
[3] The electric taste presentation device according to [1] or [2], wherein the electric signal generation unit applies the electric signal by switching at predetermined time intervals between an anodal electrical stimulation state in which the first electrode is an anode and the second electrode is a cathode, and a cathodal electrical stimulation state in which the first electrode is a cathode and the second electrode is an anode.
[4] The electric taste presentation device according to any one of [1] to [3], wherein the electric signal generation unit increases a magnitude of the electric signal from a first value to a second value by a third value at every first predetermined interval.
[5] The electric taste presentation device according to [4], wherein the electric signal generation unit increases the magnitude of the electric signal from the second value to a fourth value by a fifth value at every second predetermined interval, which is longer than the first predetermined interval.
[6] The electric taste presentation device according to any one of [1] to [5], wherein the electric signal generation unit applies the electric signal so as to stimulate a vagus nerve of the human body.
[7] The electric taste presentation device according to any one of [1] to [5], wherein the electric signal generation unit applies the electric signal so as to stimulate a muscle involved in swallowing of the human body.

According to the present invention, it is possible to suppress the reduction of the taste presentation effect for a user.

### Brief Description of Drawings

FIG. 1 is a diagram showing a state in which electric taste is presented by electrical stimulation to a human body.
FIG. 2 is a diagram showing a configuration of a mask provided with the electric taste presentation device.
FIG. 3 is a block diagram showing a functional configuration of the electric taste presentation device according to an embodiment of the present invention.

### Description of Embodiments

Hereinafter, embodiments of the present invention will be described with reference to the drawings. The features shown in the embodiments described below can be combined with each other. Furthermore, an invention can be established independently for each feature.

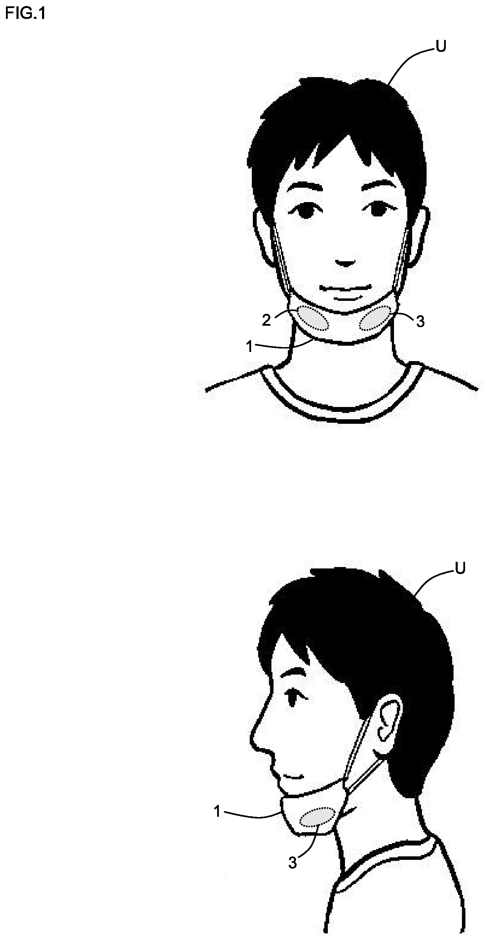

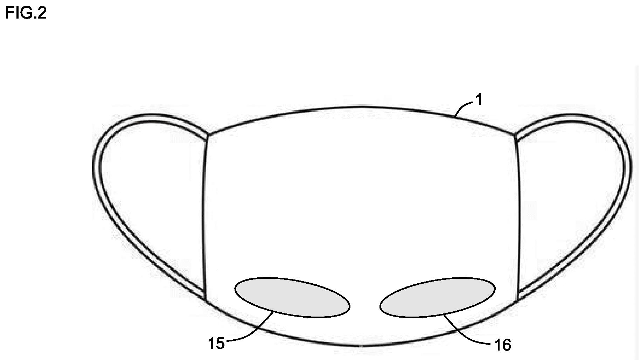

FIG. 1 is a diagram showing a state in which the electric taste presentation device 10 is used to present electric taste by electrical stimulation to a human body (hereinafter referred to as "a user U"). The electric taste presentation device 10 is provided, for example, on a mask 1 that can be worn by hooking straps on the user U's right and left ears to cover the user U's jaw (the central part of the mandible, which is the center of the face below the mouth). The electric taste presentation device 10 applies an electric signal between an elongated first electrode 15 (see FIG. 2) that comes into contact with an application site 2 (corresponding to the "first portion" of the present invention) on the user U's jaw and an elongated second electrode 16 (see FIG. 2) that comes into contact with an application site 3 (corresponding to the "second portion" of the present invention) on the user U's jaw, thereby forming a closed circuit around the jaw area and consequently applying the electric signal to the user U's taste organs to present electric taste to the user U.

FIG. 2 is a diagram showing the configuration of the mask 1 provided with the electric taste presentation device 10. As shown in FIG. 2, on the back side (the side that is worn against the user U's face) of the mask 1, an elongated first electrode 15 is attached so as to come into contact with the application site 2 on the user U's jaw, and an elongated second electrode 16 is attached so as to come into contact with the application site 3 on the user U's jaw.

In this embodiment, the application site 2 is the front or rear surface of the user U's right jaw. The application site 3 is the front or rear surface of the user U's left jaw. Note that the application site 2 may be the front or rear surface of one of the upper and lower parts of the user U's right jaw, and the application site 3 may be the front or rear surface of the other of the upper and lower parts of the user U's right jaw. Furthermore, the application site 2 may be the front or rear surface of one of the upper and lower parts of the user U's left jaw, and the application site 3 may be the front or rear surface of the other of the upper and lower parts of the user U's left jaw.

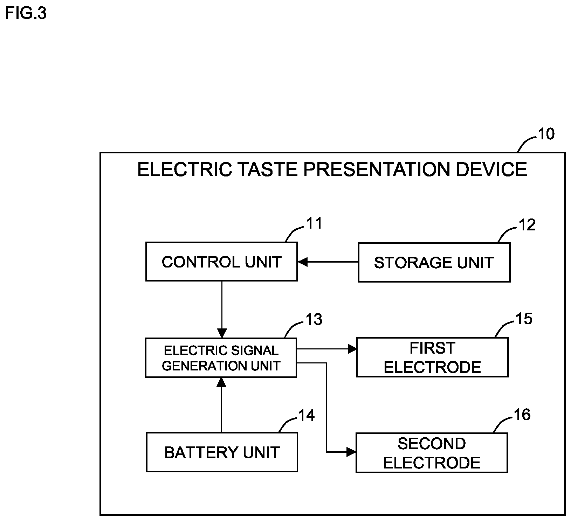

FIG. 3 is a block diagram showing a functional configuration of the electric taste presentation device 10 according to an embodiment of the present invention. As shown in FIG. 3, the electric taste presentation device 10 includes a control unit 11, a storage unit 12, an electric signal generation unit 13, a battery unit 14, a first electrode 15, and a second electrode 16.

The control unit 11 is, for example, a CPU (Central Processing Unit), a microprocessor, a DSP (Digital Signal Processor), or the like, and controls the overall operation of the electric taste presentation device 10.

A part of the storage unit 12 is configured by, for example, RAM (Random Access Memory), DRAM (Dynamic Random Access Memory), or the like, and is used as a work area or the like when the control unit 11 executes processing based on various programs.

Another part of the storage unit 12 is, for example, a non-volatile memory such as ROM (Read Only Memory) or an HDD (Hard Disk Drive), and stores various data and programs used for processing by the control unit 11. The storage unit 12 can hold a database including one or more tables for recording various information and processing results.

The programs stored in the storage unit 12 are, for example, an OS (Operating System) for realizing the basic functions of the electric taste presentation device 10, drivers for controlling various hardware, and programs for realizing various functions.

The electric signal generation unit 13 applies an electric signal between the first electrode 15 and the second electrode 16 under the control of the control unit 11. For example, the electric signal generation unit 13 sets the first electrode 15 as an anode and the second electrode 16 as a cathode, and applies an electric signal between the first electrode 15 and the second electrode 16 (anodal electrical stimulation state). Alternatively, the electric signal generation unit 13 sets the first electrode 15 as a cathode and the second electrode 16 as an anode, and applies an electric signal between the first electrode 15 and the second electrode 16 (cathodal electrical stimulation state). When an electric signal is applied between the first electrode 15 and the second electrode 16, a taste-adjusting current flows through the taste organs in the oral cavity due to the applied electric signal, thereby enhancing, suppressing, or presenting taste in the oral cavity.

The electric signal generation unit 13 generates electric signals of various waveforms by combining the amplitude, frequency, waveform, intermittent duration, and the like of the electric signal to induce or suppress taste.

The electric signal generation unit 13 can enhance or suppress saltiness, sourness, bitterness, sweetness, and umami by variously combining the amplitude, frequency, waveform, intermittent duration, etc., of the electric signal. Furthermore, the electric signal generation unit 13 can present taste by individually enhancing or suppressing each of saltiness, sourness, bitterness, sweetness, and umami.

In addition, the electric signal generation unit 13 can provide "deliciousness" obtained from meals by enhancing or suppressing astringency, spiciness, fatty taste, harshness, a cooling sensation, richness, and thickness, and by presenting the spread and duration of these tastes, by variously combining the amplitude, frequency, waveform, intermittent duration, etc., of the electric signal.

In this embodiment, by bringing the elongated first electrode 15 into contact with the application site 2 on the user U's jaw and the elongated second electrode 16 into contact with the application site 3 on the user U's jaw, the application range of the electric signal to the jaw is dispersed when applying electrical stimulation to the user U's taste organs in the oral cavity. This suppresses the risk of the user U experiencing pain or discomfort, and consequently suppresses the reduction of the taste presentation effect for the user U.

Note that, from the viewpoint of reducing the bias of locations where the user U experiences pain or discomfort, the electric signal generation unit 13 may apply the electric signal by switching at predetermined time intervals (for example, several milliseconds) between the anodal electrical stimulation state in which the first electrode 15 is the anode and the second electrode 16 is the cathode, and the cathodal electrical stimulation state in which the first electrode 15 is the cathode and the second electrode 16 is the anode.

Furthermore, from the viewpoint of further alleviating skin discomfort and pain experienced by the user U by gradually increasing the magnitude of the electric signal (that is, increasing the electric signal while allowing the user to gradually get used to the skin discomfort and pain), the electric signal generation unit 13 may increase the magnitude of the electric signal from a first value (e.g., 0.5 mA) to a second value (e.g., 1.0 mA) by a third value (e.g., 0.1 mA) at every first predetermined interval (e.g., 5 seconds). In this case, the electric signal generation unit 13 may increase the magnitude of the electric signal from the second value (e.g., 1.0 mA) to a fourth value (e.g., 1.5 mA) by a fifth value (e.g., 0.1 mA) at every second predetermined interval (e.g., 10 seconds), which is longer than the first predetermined interval (e.g., 5 seconds).

Furthermore, from the viewpoint of further alleviating skin discomfort and pain experienced by the user U, the electric signal generation unit 13 may stop the application of the electric signal for a second predetermined period (e.g., 1 minute) every time it applies the electric signal for a first predetermined period (e.g., 3 minutes).

The battery unit 14 stores, for example, electric power to be supplied to each unit of the electric taste presentation device 10 (e.g., the control unit 11 and the electric signal generation unit 13). The battery unit 14 is, for example, repeatedly dischargeable and chargeable. Note that the electric taste presentation device 10 may receive power supply from an external power source via a power cord instead of or in addition to the battery unit 14. In this case, the power cord is not particularly limited and may be, for example, a cord with a USB (Universal Serial Bus) terminal.

As described in detail above, in this embodiment, the electric taste presentation device 10 includes the elongated first electrode 15 that comes into contact with the first portion (application site 2) of the jaw of the human body (user U), the elongated second electrode 16 that comes into contact with the second portion (application site 3) of the jaw of the human body (user U), and the electric signal generation unit 13 that applies an electric signal between the first electrode 15 and the second electrode 16. Then, the electric taste presentation device 10 presents electric taste to the human body (user U) by applying the electric signal to the taste organs of the human body (user U) via the first electrode 15 and the second electrode 16.

According to this embodiment configured as described above, when applying electrical stimulation to the user U's taste organs in the oral cavity, the application range of the electric signal to the jaw is dispersed, thereby suppressing the risk of the user U experiencing pain or discomfort, and consequently suppressing the reduction of the taste presentation effect for the user U.

In the above embodiment, an example was described in which the elongated first electrode 15 comes into contact with the application site 2 on the user U's jaw, and the elongated second electrode 16 comes into contact with the application site 3 on the user U's jaw, but the present invention is not limited to this. For example, a plurality of first electrodes 15 may be separately brought into contact with the application site 2 on the user U's jaw, and a plurality of second electrodes 16 may be separately brought into contact with the application site 3 on the user U's jaw. Even in this case, when applying electrical stimulation to the user U's taste organs in the oral cavity, the application range of the electric signal to the jaw can be dispersed, thereby suppressing the risk of the user U experiencing pain or discomfort, and consequently suppressing the reduction of the taste presentation effect for the user U.

Furthermore, in the above embodiment, the electric signal generation unit 13 may apply the electric signal so as to stimulate the user U's vagus nerve. In this case, it is sufficient that the first electrode 15 and the second electrode 16 can be brought into contact with positions on the jaw that stimulate the user U's vagus nerve while applying an electric signal to the user U's taste organs. When the user U's vagus nerve is stimulated, in addition to the effect of presenting electric taste to the user U, effects such as a pain relief effect (e.g., relief of headache, lower back pain, shoulder stiffness, and improvement of kidney disease) and an obesity mitigation effect due to the stimulatory action on the satiety center can be obtained. Note that the electric signal generation unit 13 may apply the electric signal so as to stimulate the user U's muscles involved in or related to headache, lower back pain, and shoulder stiffness, or the trigeminal nerve, in order to obtain the relief effects for headache, lower back pain, and shoulder stiffness.

Furthermore, in the above embodiment, the electric signal generation unit 13 may apply the electric signal so as to stimulate the user U's muscles involved in swallowing. In this case, it is sufficient that the first electrode 15 and the second electrode 16 can be brought into contact with positions on the jaw that stimulate the user U's muscles involved in swallowing while applying an electric signal to the user U's taste organs. When the user U's muscles involved in swallowing are stimulated, in addition to the effect of presenting electric taste to the user U, an effect of inducing swallowing movement (an effect of improving dysphagia) can be obtained. Note that the electric signal generation unit 13 may apply the electric signal so as to stimulate the user U's superior laryngeal nerve (sensory nerve) in order to obtain the effect of inducing swallowing movement (the effect of improving dysphagia).

Although various embodiments of the present invention have been described above, they are presented as examples and are not intended to limit the scope of the invention. The novel embodiments can be implemented in various other forms, and various omissions, substitutions, and changes can be made without departing from the gist of the invention. The embodiments and their modifications are included in the scope and gist of the invention and are also included in the invention described in the claims and the scope of equivalents thereof.

### Reference Signs List

1: mask, 2, 3: application site, 10: electric taste presentation device, 11: control unit, 12: storage unit, 13: electric signal generation unit, 14: battery unit, 15: first electrode, 16: second electrode

## Claims

1. An electric taste presentation device for presenting electric taste by electrical stimulation to a human body, comprising:
an elongated first electrode that comes into contact with a first portion of a jaw of the human body;
an elongated second electrode that comes into contact with a second portion of the jaw of the human body; and
an electric signal generation unit that applies an electric signal between the first electrode and the second electrode,
wherein the electric taste presentation device presents electric taste to the human body by applying the electric signal to taste organs of the human body via the first electrode and the second electrode.

2. An electric taste presentation device for presenting electric taste by electrical stimulation to a human body, comprising:
a plurality of first electrodes that come into contact with a first portion of a jaw of the human body;
a plurality of second electrodes that come into contact with a second portion of the jaw of the human body; and
an electric signal generation unit that applies an electric signal between the plurality of first electrodes and the plurality of second electrodes,
wherein the electric taste presentation device presents electric taste to the human body by applying the electric signal to taste organs of the human body via the plurality of first electrodes and the plurality of second electrodes.

3. The electric taste presentation device according to claim 1 or 2,
wherein the electric signal generation unit applies the electric signal by switching at predetermined time intervals between an anodal electrical stimulation state in which the first electrode is an anode and the second electrode is a cathode, and a cathodal electrical stimulation state in which the first electrode is a cathode and the second electrode is an anode.

4. The electric taste presentation device according to claim 1 or 2,
wherein the electric signal generation unit increases a magnitude of the electric signal from a first value to a second value by a third value at every first predetermined interval.

5. The electric taste presentation device according to claim 4,
wherein the electric signal generation unit increases the magnitude of the electric signal from the second value to a fourth value by a fifth value at every second predetermined interval, which is longer than the first predetermined interval.

6. The electric taste presentation device according to claim 1 or 2,
wherein the electric signal generation unit applies the electric signal so as to stimulate a vagus nerve of the human body.

7. The electric taste presentation device according to claim 1 or 2,
wherein the electric signal generation unit applies the electric signal so as to stimulate a muscle involved in swallowing of the human body.
